**Europäisches Patentamt**

**European Patent Office**

**Office européen des brevets**

(19)

(11) Veröffentlichungsnummer: **0 199 261**
**A2**

(12) # EUROPÄISCHE PATENTANMELDUNG

(21) Anmeldenummer: 86105184.5

(22) Anmeldetag: 15.04.86

(51) Int. Cl.⁴: **G 01 N 33/564**
**G 01 N 33/543**

(30) Priorität: 17.04.85 DE 3513915

(43) Veröffentlichungstag der Anmeldung:
29.10.86 Patentblatt 86/44

(84) Benannte Vertragsstaaten:
AT BE CH DE FR GB IT LI LU NL SE

(71) Anmelder: Heyl Chemisch-pharmazeutische Fabrik
GmbH & Co. KG
Goerzallee 253
D-1000 Berlin 37(DE)

(72) Erfinder: Bunte, Thomas, Dr.
Gustav-Müller-Strasse 31
D-1000 Berlin 62(DE)

(72) Erfinder: Ruprecht, Johann, Dr. Dipl.-Chem.
Rheinstrasse 2-3
D-1000 Berlin 41(DE)

(72) Erfinder: Vorlaender, Wolfgang, Dr. med. Dipl.-Chem.
Otto-Erich-Strasse 12
D-1000 Berlin 39(DE)

(72) Erfinder: v. Kleist, Elke, Dr.
Neue Kreisstrasse 12
D-1000 Berlin 39(DE)

(72) Erfinder: Schmolke, Bernd, Dr. Dipl.-Chem.
Palmzeile 17
D-1000 Berlin 38(DE)

(72) Erfinder: Heyl, Eduard, Dr. med.
Limastrasse 11
D-1000 Berlin 45(DE)

(72) Erfinder: Parr, Wolfgang, Dr. Dipl.-Chem.
Eitel-Fritz-Strasse 16
D-1000 Berlin 45(DE)

(74) Vertreter: Kinzebach, Werner, Dr. et al,
Patentanwälte Reitstötter, Kinzebach und Partner
Sternwartstrasse 4 Postfach 86 06 49
D-8000 München 86(DE)

(54) Immunologischer Testkit.

(57) Die Erfindung betrifft einen immunologischen Testkit für
die Diagnostik rheumatischer Erkrankungen, umfassend
a) einen Träger, auf dem Kollagen als Antigen immobilisiert ist
b) ein enzymmarkiertes Antiantikörper-Reagenz
c) ein Substrat, das zur enzymkatalysierten Farbstoffbildung befähigt ist.

Die Erfindung betrifft einen immunologischen Testkit für den Nachweis von Autoantikörpern gegen Kollagen im Serum und in Synovialflüssigkeiten. Dieser Nachweis von Antikörpern gegen körpereigene Kollagene, also von Autoantikörpern, erlaubt Aussagen für die Diagnostik von Erkrankungen, insbesondere denen des rheumatischen Formenkreises (z.B. chronische Polyarthritis, rheumatoide Arthritis, Lupus erythematodes, Sjörgen Syndrom, Morbus Bechterew, Sklerodermie u.a.)

Ein bis heute übliches Nachweisverfahren für Autoantikörper ist der Antiglobulin-Konsumptionstest (AGK-Test). Diese Methode ist technisch und in der Durchführung sehr aufwendig. Sie führt deshalb oft zu wenig vertrauenswürdigen Ergebnissen.

Weitere derzeit durchgeführte Rheumateste beruhen auf dem Nachweis von Rheumafaktoren (RF). Dabei handelt es sich um eine heterogene Gruppe von Autoantikörpern gegen verschiedene körpereigene Gammaglobuline. Allerdings treten diese Rheumafaktoren nicht bei allen rheumatischen Affektionen auf (seropositiv / seronegativ). Dagegen lassen sie sich auch bei Gesunden und bei nichtrheumatischen Erkrankungen nachweisen. Zur diagnostischen Bestimmung der Rheumafaktoren sind eine Vielzahl von qualitativen und quantitativen Methoden bekannt, die oft sehr aufwendig und wenig spezifisch sind (Waaler-Rose-Test, Waller-Test, Latex-Fixationstest, passiver Hämaglutinationstest (K. Fehr, A. Böni, "Gelenke - Chronische Polyarthritis", in "Immunologie", Herausgegeben von K.O. Vorländer, 1983, S. 465-499)).

Zur Differentialdiagnose werden, insbesondere bei chronischer Polyarthritis (cP), antinukleäre Faktoren, Autoantikörper gegen Zellkernbestandteile (DNS, Histone, Nukleolus-RNS u.a.) bestimmt. Auch Teste auf C-reaktives Protein und zur Bestimmung des Antistreptolysin-Titers werden in der Rheumadiagnostik eingesetzt.

Es ist aus der Literatur bekannt, daß bei Erkrankungen des rheumatischen Formenkreises sowohl im Serum als auch in Synovialflüssigkeiten Autoantikörper gegen körpereigene Kollagene auftreten (C.Steffen et al., Klin. Wschr. 51, 222-229 (1973); C. Steffen, Z. Rheumatol. 37, 131-147 (1978)). Diese Autoantikörper stellen deshalb einen medizinisch interessanten Diagnoseparameter dar.

Der Erfindung liegt die Aufgabe zu Grunde, einen immunologischen Testkit für die Diagnostik von Erkrankungen des rheumatischen Formenkreises bereitzustellen, bei dem ein Assay in technisch einfach durchführbarer Form ablaufen kann. Die erforderlichen Mengen an Serum oder Synovialflüssigkeiten sollen gering sein. Die Sensitivität des Nachweises muß möglichst hoch und die Spezifität direkt überprüfbar sein. Außerdem soll sowohl eine einfache qualitative wie auch eine quantitative Analyse mit Hilfe dieses Assays möglich sein.

Diese Aufgabe wird gelöst mit einem immunologischen Testkit der zuvor genannten Art, umfassend

   a) einen Träger, auf dem das Kollagen immobilisiert ist,

   b) ein enzymmarkiertes Antiantikörper-Reagenz

   c) ein Substrat, das zur enzymkatalysierten Farbstoffbildung befähigt ist.

Der erfindungsgemäße immunologische Testkit basiert auf
der folgenden Testmethode:

a) Ein Antigen (Kollagen) wird auf einem geeigneten
Trägermaterial adsorbiert und immobilisiert.

b) Im Serum oder der Synovialflüssigkeit der Patienten enthaltene Autoantikörper werden an das immobilisierte Antigen (Kollagen) gebunden.

c) Der an das Antigen gebundene Autoantikörper wird
nachgewiesen.

Erfindungsgemäß ist der immunologische Testkit für verschiedene Testvarianten geeignet. Es können unterschiedliche Kollagene auf den Trägern immobilisiert sein. Besonders bevorzugt sind menschliche Kollagene der Typen
I bis VI. Auch Kalbskollagen (überwiegend Typ I) läßt
sich einsetzen. Eine Untersuchung, gegen welchen Kollagentyp der Autoantikörper gerichtet ist, erlaubt zusätzliche Aussagen für die medizinische Diagnostik.

Als Träger im erfindungsgemäßen immunologischen Testkit
besonders geeignet sind käufliche Mikrotiterplatten, (z.B.
der Firmen Greiner, Nunc, Dynatech, Miles, Millipore).
Diese bestehen vorzugsweise aus einem Polystyrolmaterial.
Aber auch kommerziell erhältliche Spezialröhrchen sind
einsetzbar. Auch Trägermaterialien aus Nitrocellulose,
oder anderen geeigneten Materialien, welche befähigt sind,
das Antigen zu binden, können verwandt werden, z.B. Diazobenzyloxymethyl- (DBM) oder Diazophenylthioethermaterialien (DPT). Für die Durchführung des ELISA-Verfahrens
eignen sich alle im Handel erhältlichen Träger, für die
Blotingverfahren sind insbesondere die zuletzt genannten Träger bevorzugt.

Der Nachweis des an das immobilisierte Antigen gebundenen Autoantikörpers erfolgt mittels eines Antiantikörpers, der zusätzlich mit einem Enzym versehen ist. Dieses Enzym katalysiert die Umwandlung zunächst farbloser Substratmoleküle in ein farbiges Endprodukt. Der hierbei gebildete Farbstoff kann visuell bestimmt werden, wodurch man eine rein qualitative Ja-Nein-Aussage erhält. Er kann aber auch durch geeignete Meßverfahren quantitativ bestimmt werden, wodurch man eine semi-quantitative oder quantitative Aussage erhält.

Erfindungsgemäß ist der immunologische Testkit für verschiedene Testvarianten geeignet. So kommen für die Art des Nachweissystems der Enzyme-Linked-Immuno-Sorbent-Assay (ELISA) ebenso wie der Proteinblotassay und der Proteindotblotassay in Betracht.

Der ELISA beruht auf folgenden Prinzipien (E. Engvall; Methods in Enzymology 70, 419-439 (1980)): Ein Antigen wird an einer geeigneten Oberfläche adsorbiert. Das immobilisierte Antigen wird mit den Lösungen, die auf Antikörper getestet werden sollen, inkubiert. Dabei binden eventuell vorhandene Antikörper an das Antigen. Mit Hilfe eines enzymmarkierten Antiantikörpers, der wiederum den gebundenen Antikörper erkennt, läßt sich dann der gegen das Antigen gerichtete Antikörper mit einer Enzymreaktion durch Farbumschlag nachweisen. Parallel durchgeführte positive und negative Kontrollen garantieren die Richtigkeit des Nachweises.

Die ELISA-Methode gestattet jedoch nicht direkt zu sehen, gegen welches Antigen ein Antikörper gerichtet ist und läßt deshalb nur bestimmte Aussagen über die Spezifität der Nachweisreaktion zu. Mit Hilfe der Proteinblottechnik kann jedoch die im ELISA gemessene Reaktion hinsichtlich ihrer Spezifität genau untersucht werden. Dabei werden Proteine mittels Polyacrylamidgelelektrophorese nach ihrem Molekulargewicht aufgetrennt und danach elektrophoretisch auf Nitrozellulosepapier transferiert, an das sie mit hoher Affinität binden. Die übrigen freien Bindungsstellen auf der Nitrozellulose werden anschließend mit einer ein Trägerprotein (beispielsweise Rinderserum-Albumin) enthaltenden Lösung abgesättigt. Danach wird die Nitrozellulose in einer antikörperhaltigen Lösung inkubiert. Dabei binden die Antikörper an das Antigen. Mit Hilfe des bereits genannten enzymmarkierten Antiantikörpers und eines präzipitierbaren Enzymsubstrates wird das Antigen in einer Farbreaktion nachgewiesen.

Im Proteindotblotassay wird das Protein ohne vorherige geleketrophoretische Auftrennung direkt auf Nitrocellulose, an das alle Proteine stark binden, adsorbiert. Anschließend wird wie beim Proteinblotassay weiterverfahren. Das Substrat kann so gewählt werden, daß es bei der enzymkatalysierten Umsetzung einen wasserlöslichen oder einen wasserunlöslichen, präzipitierenden Farbstoff ergibt. Die positive Reaktion ist dann entweder am Farbumschlag der Substratlösung oder an dem sich anfärbenden Proteinfleck auf dem Papier zu erkennen (Teststreifen zur einfachen Ja-Nein-Aussage):

Erfindungsgemäß sind auch Blot-Varianten brauchbar. Hierbei wird ein Teststreifen mit immobilisiertem Antigen, daran gebundenem Autoantikörper und angelagertem enzymmarkiertem Antiantikörper in eine geeignete Substratlösung eingebracht. Hierbei entsteht ein löslicher Farbstoff, welcher sich nicht an den Teststreifen anlagert. Die entstandene Farbstofflösung kann anschließend problemlos quantitativ ausgewertet werden.

Das Substrat für die enzymkatalysierte Farbstoffreaktion und damit der resultierende Farbstoff läßt sich variieren. Bevorzugt sind Substrate, welche zur Bildung löslicher Farbstoffe befähigt sind, z.B. 3, 3', 5, 5'-Tetramethylbenzidin, 0,004 % $H_2O_2$ in 100 mM Natriumacetat pH 6 bei Verwendung von Meerettichperoxidase oder vorzugsweise 0,1 % p-Nitrophenylphosphat, 10 % Diethanolamin-HCl, pH 9,8, 5 mM $MgCl_2$ bei Verwendung von alkalischer Phosphatase. In gleicher Weise sind Substrate geeignet, welche präzipitierende Farbstoffe bilden, z.B. 10 % Diethanolamin-HCl, pH 9,8, 0,01 % Nitroblautetrazolium, 0,005 % 5-Brom-4-chlor-3-indolylphosphat, 4 mM MgCl; 0,25 mg/ml 3, 3'-Diaminobenzidin, 0,015 % $H_2O_2$ in 50 mM Tris-HCl pH 7. Die verschiedenen eingesetzten Substrate sind alle im Handel erhältlich.

Lösliche Farbstoffe bevorzugt man normalerweise bei der ELISA-Technik, da diese photometrisch leicht quantitativ bestimmt werden können. Bei den Blottechniken verwendet man dagegen nichtlösliche Farbstoffe, so daß die einzelnen "Banden" erkennbar bleiben, und sich der Farbstoff nicht auf dem gesamten Träger verteilt. Quantitative Bestimmungen sind in diesem Fall apparativ aufwendiger.

Vom Immunsystem werden grundsätzlich zunächst Antikörper der Klasse IgM gebildet, welche dann durch IgG-Antikörper ersetzt werden. Mit Hilfe entsprechender Antiantikörper (Anti-IgG, Anti-IgM) kann die Immunglobulinklasse des Autoantikörpers bestimmt werden. Möglicherweise lassen sich damit Aussagen über den Krankheitsverlauf gewinnen. Antiantikörper, gegen menschliches IgG ( γ-kettenspezifisch; $IgG_{1-4}$) bzw. IgM (µ-kettenspezifisch)sind im Handel erhältlich, z.B. von Sigma, Dako, Behringwerken und Boehringer, Mannheim.

Bei den Testsubstanzen für positive und negative Kontrollen handelt es sich um die auf dem vorliegenden Gebiet üblichen Substanzen. Insbesondere wird als negative Kontrolle bei den hier verwendeten Testsystemen ein menschliches Serum benutzt, das keine Antikörper gegen Kollagene enthält, so daß damit kein Farbumschlag stattfinden sollte.

Als positive Kontrolle dient ein Gemisch aus Seren von Patienten, das Autoantikörper enthält. Dieses Serumgemisch ist so eingestellt, daß es einen definierten Farbumschlag verursacht. Dieser Farbumschlag dient dann als Referenzwert für die zu messenden Seren mit unbekanntem Gehalt an Autoantikörpern.

Man kann den Träger mit beliebigen Mengen Kollagen versehen, bei Verwendung von Mikrotiterplatten ist es bevorzugt, mindestens 400 ng Protein pro Kavität, vorzugsweise 400 ng - 1000 ng Protein/Kavität einzusetzen.

Die Herstellung und Anwendung eines erfindungsgemäßen Testsystems erfolgt folgendermaßen:

Zum Beschichten eines Trägers mit Kollagenen wird dieser in einer Lösung von Kollagenen in einem Puffer 8 bis 16 Stunden, vorzugsweise 12 Stunden, bei 37°C inkubiert. Für diesen Zweck geeignete Puffer sind beispielsweise 15 mM $Na_2CO_3$, 34 mM $NaHCO_3$, pH 9,6; oder 0,12 M NaCl, 0,01 M $Na_2HPO_4$, 0,004 M $KH_2PO_4$, pH 7,4.

Anschließend wäscht man die Kavitäten des Trägers mindestens einmal mit einem Waschpuffer, z.B. 0,12 m NaCl, 0,01 m $Na_2HPO_4$, 0,004 m $KH_2PO_4$, pH 7,8 (PBS-Lösung), oder 50 mM Tris-HCl, pH 7,4, 100 mM NaCl.

Die Waschpufferlösungen enthalten vorzugsweise Tween 20 [R], beispielsweise 0,05 Gew.-%. Durch Zugabe von Natriumchlorid läßt sich die Wirkung der Waschpuffer noch verbessern. Tween 20 [R] kann beispielsweise durch 0,1 % NP40, 0,1 % Triton X-100 oder Brij 59 ersetzt werden. Der zuerst genannte Puffer ist bevorzugt.

Nach dem Waschvorgang erfolgt eine mehrstündige, vorzugsweise 1- bis 4-stündige, Inkubation mit Patientenseren bei Raumtemperatur. Die Patientenseren werden in einem Puffer verdünnt angewandt, die Verdünnung liegt bei 1:10 - 1:2000, vorzugsweise bei 1:20 - 1:100. Geeignete Verdünnungspuffer sind die oben genannten Waschpuffer in Verbindung mit 1 % Rinderserum-Albumin oder 10 % fötales Kälberserum, 100 mM NaCl, 10 mM Tris-HCl, pH 7,4; oder 3 % Gelatine, 100 mM NaCl, 10 mM Tris-HCl, pH 7,4. Nach der Inkubation wäscht man erneut mit einem der oben genannten Waschpuffer.

Anschließend erfolgt eine mehrstündige, vorzugsweise 2-stündige, Inkubation mit einem in einem Puffer verdünnten Antiantikörperkonjugat, z.B. Anti-human-IgG bzw. -IgM konjugiert mit alkalischer Phosphatase oder α-Human-IgG bzw. -IgM konjugiert mit Meerettichperoxidase. Die Inkubation erfolgt bei Raumtemperatur, die Konjugatverdünnung beträgt 1:250 - 1:1000, vorzugsweise 1:400 - 1:600, am bevorzugtesten 1:500.

Man wäscht erneut mit einem der vorgenannten Waschpuffer. Daran anschließend erfolgt die Entwicklung der Reaktion, wozu man die oben genannten Substrate und Farbstoffe verwendet. Dabei erfolgt die Inkubation der Substrate und Farbstoffe, die in 10 % Diethanolamin-HCl pH 9,8; 5 mM $NaCL_2$ gelöst wurden, mit dem sich in den vorhergehenden Schritten gebildeten Antigen-Antikörperkomplexen für 20 - 30 Minuten bei 37°C.

Mit dem erfindungsgemäßen Testkit lassen sich der Nachweis von Autoantikörpern gegen Kollagen und die Titerbestimmungen, beispielsweise mit der ELISA-Technik, einfach und gut reproduzierbar durchführen. Man kann mit diesem Test zwischen Immunglobulinen der Klasse IgG und IgM unterscheiden. Durch Proteinblottechnik kann die Spezifität dieser Arbeitsweise direkt überprüft werden.

Der erfindungsgemäße Testkit ist auch zum schnellen Nachweis von Autoantikörpern mit einem Teststreifen geeignet. Hierbei erreicht man eine Ja-Nein-Aussage. Der Testkit ist jedoch auch für semi-quantitative und quantitative Bestimmungen hervorragend geeignet.

## Beispiel 1

Es wurde ein erfindungsgemäßes Testsystem hergestellt. Hochreine menschliche Kollagene (Typ I - VI), die sich in Puffer A (15 mM $Na_2CO_3$ ; 34 mM $NaHCO_3$; pH 9,6) befanden, wurden für 12 Stunden bei 37°C in Greiner-Mikrotiterplatten (Mikrotiterplatte, F-Form 655101) inkubiert. Anschließend wurden die Kavitäten der Mikrotiterplatte 3-fach mit Puffer B (0,12 M NaCL, 0,01 M $Na_2HPO_4$ ; 0,004 M $KH_2PO_4$; 0,05 % Tween $20^R$; pH 7,8) gewaschen. Auf den Waschvorgang folgte für zwei Stunden die Inkubation mit Patientenseren, verdünnt in Puffer C (Puffer B, 1 % Rinderserum-Albumin) bei Raumtemperatur. Nach anschließender Wäsche der Kavitäten mit Puffer B erfolgte die zweistündige Inkubation mit dem in Puffer C verdünnten Antiantikörperkonjugat (Hier Anti-human-IgG bzw. -IgM konjugiert mit alkalischer Phosphatase) bei Raumtemperatur. Nach erneuter Wäsche wurde die Reaktion mit Puffer D (0,1 % p-Nitrophenylphosphat; 10 % Diethanolamin-HCl; pH 9,8; 5 mM $MgCl_2$) entwickelt. Sowohl die Menge des an die Kavität adsorbierten Kollagens als auch die Verdünnung des Antiantikörperkonjugats sind entscheidende Parameter für die Nachweisreaktion. Es konnte nachgewiesen werden, daß 400 ng an die Kavität adsorbiertes Kollagen und eine Konjugatverdünnung von 1:500 in dem hier beschriebenen Testsystem zu guten Ergebnissen führen.

## Beispiel 2

Die in Beispiel 1 beschriebenen Teste auf Autoantikörper gegen Kollagene menschlichen Ursprungs lassen sich auch mit dem unter 1 beschriebenen Protokoll im Polystyrolröhrchen (z.B. Nunc Star Tubes) durchführen. Die Ergebnisse sind mit denen in Beispiel 1 genannten identisch.

## B e i s p i e l   3

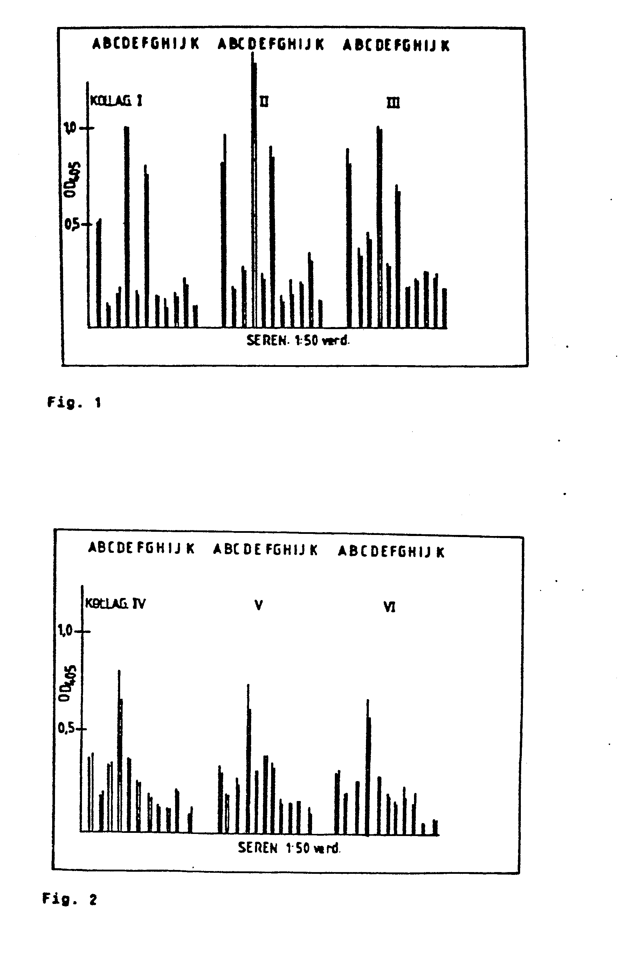

Nach der Herstellung des ELISA-Protokolls unter der Verwendung des Testsystems gemäß Beispiel 1 wurden Humanseren auf Autoantikörper gegen die verschiedenen Kollagene I bis VI getestet. Die Serumverdünnung betrug hierbei 1:50. Die Ergebnisse der Versuche sind in den Fig. 1 und 2 dargestellt. Es wurden Doppelbestimmungen durchgeführt, um den Assay hinsichtlich seiner Reproduzierbarkeit überprüfen zu können. Es zeigten sich signifikante Unterschiede in den Reaktionen der einzelnen Patienten gegen die menschlichen Kollagene I, II und III (Fig.1). Gegen die Kollagene IV, V und VI waren die Reaktionen schwächer (Fig.2). Als positive Kontrolle werden bei diesen Experimenten die Antikörper einzelner Patienten gegen Bakterienmembranen gemessen, als negative Kontrolle die Reaktionen gegen Rinderserum-Albumin und die Reaktionen mit normalem Patientenserum gegen die Kollagene.

Die Seren wurden gegen die Kollagene titriert. Hierbei erhielt man bei den einzelnen Patienten stark unterschiedliche Serumtiter. Es wurden hierbei logarithmische Serumverdünnungen mit Kollagen I-VI im ELISA getestet. Alle diese Experimente dienten dem Nachweis von Autoantikörpern der IgG-Klasse.

Die Höhe des Serumtiters kann für diagnostische Zwecke von Bedeutung sein. Eine Beurteilung des Standes der Erkrankung ist hiernach möglcih.

# Beispiel 4

Der Nachweis von Autoantikörpern ist nicht nur mit Hilfe menschlicher Kollagene möglich. Auch Kalbskollagene erlauben die Darstellung von Autoantikörpern in dem im Beispiel 1 beschriebenen ELISA-Testsystem. Dazu wurden 400 ng Kalbskollagen in Puffer A 12 Stunden bei 37°C in die Kavitäten der unter Beispiel 1 bezeichneten Mikrotiterplatte inkubiert und gemäß Protokoll weiterverfahren. Die so erhaltenen Ergebnisse standen in guter Übereinstimmung zu den in Beispiel 3 aufgeführten.

# Beispiel 5

Um die Spezifität der im ELISA gemessenen Reaktionen zu überprüfen, wurden Proteinblots nach der von Towbin et al. (PNAS 76, 4350-4354 (1979)) beschriebenen Verfahren durchgeführt. Der Nachweis erfolgte mit Hilfe einer Farbstofflösung (10 % Diethanolamin-HCl; pH 9,8; 0,01 % Nitroblautetrazolium; 0,005 % 5-Bromo-4-Chloro-3-indolylphosphat; 4 mM $MgCl_2$). In der Tat konnten hierbei die Autoantikörper als direkt mit der Kollagenproteinbande reagierend nachgewiesen werden. Dies ist direkter Beweis für die Richtigkeit der im ELISA gemessenen Ergebnisse.

# Beispiel 6

Es gelang auch ein Autoantikörpernachweis gegen Kollagene in Form eines Proteindotblotassays. Hierzu wurden die Kollagene direkt auf Nitrocellulosepapierstreifen punktförmig aufgetragen. Nach Absättigung der restlichen Proteinbindungsstellen der Nitrocellulose mit Hilfe einer 1%-igen Rinderserum-Albumin-Lösung wurden die Teststreifen in Serum für 30 Minuten inkubiert. Nach zweimaliger Wäsche in Puffer E (10 mM Tris-HCl; 0,9 % NaCl) wurde der Teststreifen in Konjugatlösung inkubiert und abermals gewaschen. Anschließend wurde für 5 Minuten in der in Beispiel 5 beschriebenen Farbstofflösung inkubiert. Ein positives Ergebnis zeigte sich in einer punktförmigen Anfärbung des Nitrocellulosepapiers. Als Kontrolle wurde parallel ein Teststreifen mit Normalserum inkubiert und wie oben beschrieben weiterverfahren.

## B e i s p i e l   7

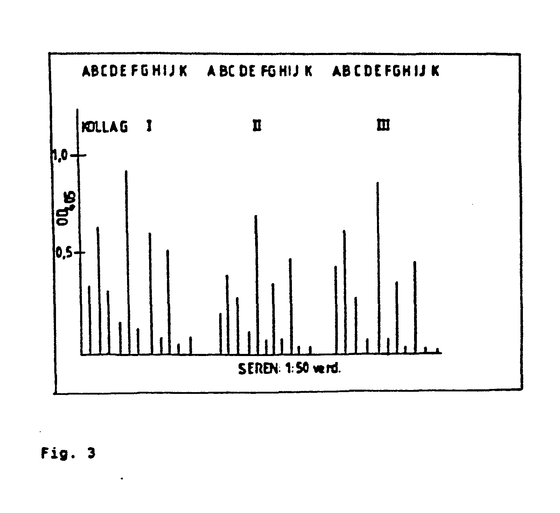

Alle bis jetzt beschriebenen Anwendungsbeispiele lassen sich problemlos auf den Nachweis von Immunglobulinen, die nicht der IgG-Klasse zugehören, übertragen. Hierzu ist es notwendig, mit einem die entsprechende Immunglobulinklasse erkennenden Antiantikörperkonjugat zu arbeiten. Hier wurden Untersuchungen mit den in Beispiel 1 bis 6 vorgestellten Methoden zum Nachweis von Autoantikörpern der IgM-Klasse durchgeführt. Die entsprechenden Ergebnisse sind in Fig. 3 dargestellt. In der Tat gibt es solche Personen, die keine gegen Kollagen gerichtete Autoantikörper der IgG-Klasse, wohl aber solche der IgM-Klasse haben.

In der nachfolgenden Tabelle sind die Ergebnisse der in Beispiel 1 bis 7 erläuterten Experimente zusammengefaßt (A-I Patientenseren; J,K   negative Kontrollseren)

## Verschiedene Screeningmethoden Auto-Ak gegen Kollagen III

| PATIENTEN | AGK | ELISA | ELISA | BLOT | BLOT | DOTBLOT |
|-----------|-----|-------|-------|------|------|---------|
|           |     | IgG   | IgM   | IgG  | IgM  | IgG     |
| A | + | +++ | ++ | +++ | ++ | ++ |
| B | + | + | +++ | +/- | +++ | - |
| C | + | + | ++ | +/- | + | - |
| D | ++ | +++ | - | +++ | - | +++ |
| E | +++ | + | +++ | +/- | ++ | - |
| F | ++ | +++ | - | ++ | - | ++ |
| G | +++ | - | ++ | - | + | - |
| H | ++ | - | - | - | - | - |
| I | +++ | - | ++ | - | ++ | - |
| J | - | - | - | - | - | - |
| K | - | - | - | - | - | - |

Patentansprüche

1. Immunologischer Testkit für den Nachweis von Autoantikörpern gegen Kollagen, umfassend

  a) einen Träger, auf dem Kollagen als Antigen immobilisiert ist

  b) ein enzymmarkiertes Antiantikörper-Reagenz

  c) ein Substrat, das zur enzymkatalysierten Farbstoffbildung befähigt ist.

2. Testkit nach Anspruch 1, dadurch gekennzeichnet, daß
er zusätzlich Testsubstanzen für eine positive und
negative Kontrolle, sowie gegebenenfalls Wasch-,
Puffer- und Verdünnungslösungen umfaßt.

3. Testkit nach Anspruch 1 oder 2, dadurch gekennzeichnet, daß es sich bei dem auf dem Träger immobilisierten Kollagen um menschliches Kollagen der Typen I
bis VI oder Kalbskollagen handelt.

4. Testkit nach einem der Ansprüche 1 bis 3, dadurch gekennzeichnet, daß das Antiantikörper-Reagenz selektiv gegen menschliche Kollagen-Autoantikörper der
Klasse IgM oder IgG aktiv ist.

5. Testkit nach einem der Ansprüche 1 bis 4, dadurch gekennzeichnet, daß das Substrat zur Bildung löslicher
oder präzipitierender Farbstoffe befähigt ist.

6. Testkit nach einem der Ansprüche 1 bis 5, dadurch gekennzeichnet, daß es sich bei dem Träger für das Antigen um eine Mikrotiterplatte, ein Röhrchen oder um einen Teststreifen aus Nitrozellulosepapier handelt.

7. Testkit nach Anspruch 6, dadurch gekennzeichnet, daß die Mikrotiterplatte 400 bis 1000 ng Kollagen pro Kavität aufweist.

8. Testkit nach einem der Ansprüche 1 bis 7 dadurch gekennzeichnet, daß es sich bei dem an den Antiantikörper gebundenen Enzym um alkalische Phosphatase handelt.

9. Testkit nach einem der Ansprüche 1 bis 8, dadurch gekennzeichnet, daß die Autoantikörper gegen Kollagen im Serum oder in Synovialflüssigkeiten nachgewiesen werden können.

Fig. 1

Fig. 2

Fig. 3